Europäisches Patentamt

European Patent Office

Office européen des brevets

(11) Veröffentlichungsnummer : **0 029 864**
B1

(12) # EUROPÄISCHE PATENTSCHRIFT

(45) Veröffentlichungstag der Patentschrift :
11.05.83

(51) Int. Cl.³ : **A 61 M 25/00**

(21) Anmeldenummer : **79104815.0**

(22) Anmeldetag : **01.12.79**

(54) **Katheteranschlusskopf mit mindestens einem Kanal in einem Grundkörper.**

(43) Veröffentlichungstag der Anmeldung :
**10.06.81 Patentblatt 81/23**

(45) Bekanntmachung des Hinweises auf die Patenterteilung : **11.05.83 Patentblatt 83/19**

(84) Benannte Vertragsstaaten :
**AT BE CH DE FR GB IT LU NL SE**

(56) Entgegenhaltungen :
**DE A 2 845 643**
**US A 4 112 932**

(73) Patentinhaber : **Intermedicat GmbH**
**Gerliswilstrasse 45**
**CH-6020 Emmenbrücke (CH)**

(72) Erfinder : **Weikl, Andreas, Dr.**
**Kosbacher Weg 51**
**D-8520 Erlangen (DE)**
Erfinder : **Hubmann, Max, Dr.**
**Ratsberger Strasse 24**
**D-8520 Erlangen (DE)**

(74) Vertreter : **Selting, Günther, Dipl.-Ing. et al**
**Deichmannhaus am Hauptbahnhof**
**D-5000 Köln 1 (DE)**

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

# Katheteranschlußkopf mit mindestens einem Kanal in einem Grundkörper

Die Erfindung bezieht sich auf einen Katheteranschlußkopf mit mindestens einem Kanal in einem Grundkörper gemäß dem Gattungsbegriff des Anspruches 1.

Bei einem bekannten Katheter dieser Art (DE-B-22 38 722) besteht das hohle Anschlußstück aus einer Katheternabe, einer Anschlußnabe für das Infusionssystem oder für ein weiteres Katheter und einem zwischen beiden Naben angeordneten elastischen Schlauchstück. Die Wandung des seitlich abbiegbaren Schlauchstückes bildet den von der Hohlnadel durchbohrbaren Abschnitt aus elastischem, selbstabschließendem Material, z. B. Gummi.

Abgesehen davon, daß beim Durchbohren der Gummiwandung mit Hilfe einer scharfen Metallkanülenspitze keine Gewähr dafür gegeben ist, daß nicht doch kleinere oder kleinste Teile der Gummiwandung abgeschabt werden und auf direktem Wege in die Blutbahn gelangen, besteht bei dieser vorbekannten Anordnung außerdem die Gefahr, daß die Gummiwandung mittels einer Metallkanüle nochmals durchbohrt wird und dabei der vom ersten Punktionsvorgang her in dem Blutgefäß zurückgebliebene Katheter bzw. die Kathetereinführröhre beschädigt werden kann.

Ausgehend von dem vorstehend geschilderten Stand der Technik liegt der vorliegenden Erfindung die Aufgabe zugrunde, einen Katheteranschlußkopf für ein Venenpunktions- und Verweilkanülenbesteck zu schaffen, der einerseits handlich und für den Arzt sicher bedienbar ist und der andererseits für den Patienten die Gefahr einer Embolie bzw. Infektion auf ein Mindestmaß herabsetzt bzw. ganz ausschaltet.

Diese Aufgabe wird bei einer Vorrichtung nach dem Oberbegriff des Anspruches 1 erfindungsgemäß durch die kennzeichnenden Merkmale des Anspruches 1 gelöst.

Dadurch, daß der Punktionskanülenkanal beim Zurückziehen der Punktionskanüle automatisch verschlossen und verriegelt wird und sowohl dieser als auch gegebenenfalls weitere Kanäle des Anschlußkopfes mit Hilfe von mechanischen Dichtungselementen gegenüber der Umwelt automatisch abgedichtet werden, können keine weiteren Punktionskanülen in den oder in die Kanäle des Grundkörpers eingeführt und damit bereits liegende Katheter nicht versehentlich beschädigt werden. Durch die Dichtungselemente ist darüber hinaus sichergestellt, daß weder Luftbläschen noch irgendwelche Schmutzteilchen in die Blutbahn gelangen können, wodurch die Gefahr von Embolien wesentlich herabgesetzt ist.

Vorteilhafte Ausgestaltungen des Erfindungsgedankens sind Gegenstand von Unteransprüchen.

Die Erfindung wird nachfolgend anhand von Ausführungsbeispielen näher erläutert, die in der Zeichnung dargestellt sind. Es zeigen :

Figur 1 in Seitenansicht einen Katheteranschlußkopf mit eingeschobener Punktionskanüle,

Figur 2 einen entsprechenden Längsschnitt hierzu und

Figur 2a in vergrößertem Maßstab die arretierte Verriegelungseinrichtung in Ansicht ;

Figur 3 in Seitenansicht einen Katheteranschlußkopf bei herausgezogener Punktionskanüle sowie eingeschwenktem Verschlußelement ;

Figur 4 einen entsprechenden Längsschnitt hierzu und

Figur 4a in vergrößertem Maßstab die freigegebene Verriegelungseinrichtung in Ansicht ;

Figur 5 einen teilweisen Schnitt des Katheteranschlußkopfes in Draufsicht ;

Figur 6 in perspektivischer Seitenansicht einen Katheteranschlußkopf mit auf dem Verschlußglied vorbereitend angebrachtem Katheter und noch außenliegender Punktionskanüle ;

Figur 7 ebenfalls in perspektivischer Seitenansicht einen Katheteranschlußkopf bei herausgezogener Punktionskanüle und eingeschwenktem Verschlußelement und dessen Verriegelungsmöglichkeit sowie die Abdichtung gegen Lufteintritt und

Figur 7a in vergrößertem Maßstabe den Aufbau der Verriegelung ;

Figur 8 in Seitenansicht einen Längsschnitt durch einen Katheteranschlußkopf mit innenliegendem Verschlußelement sowie dessen Verriegelung ;

Figur 9 einen unter der Wirkung einer Feder stehenden Schieber in der Stellung, in der nur die Einführungsöffnung für die Punktionskanüle offen ist,

Figur 9a die Stellung des Schieber, in der die Einführung einer Punktionskanüle nicht mehr möglich ist und

Figur 9b in vergrößertem Maßstab eine Teilansicht der beiden Endstellungen des Schiebergestänges ;

Figur 10 ein Verschlußglied als drehbare Scheibe mit einer exzentrisch angebrachten Bohrung, die in der Einführlage für die Punktionskanüle steht,

Figur 10a die Stellung des scheibenförmigen Verschlußgliedes bei gesperrter Einführöffnung für die Punktionskanüle und den geöffneten Kanal für die Einführung eines Katheters oder für den Anschluß eines Infusionssystems und

Figur 10b in vergrößertem Maßstab in Ansicht die Verriegelung in der Sperrlage.

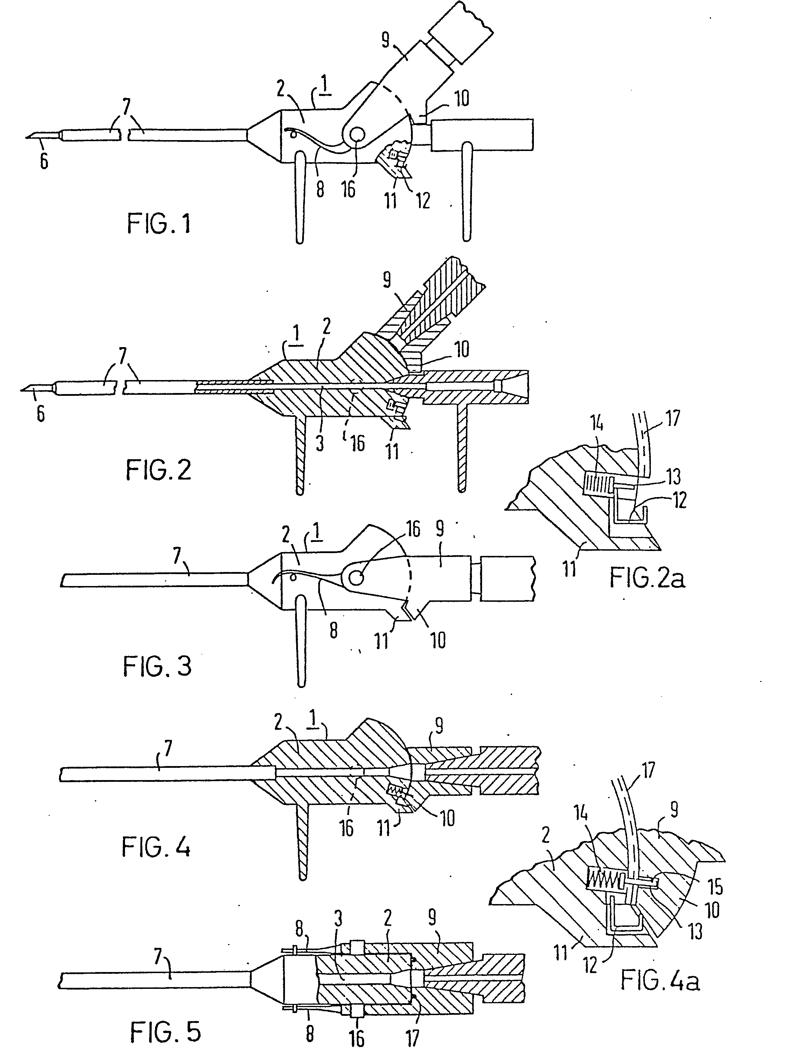

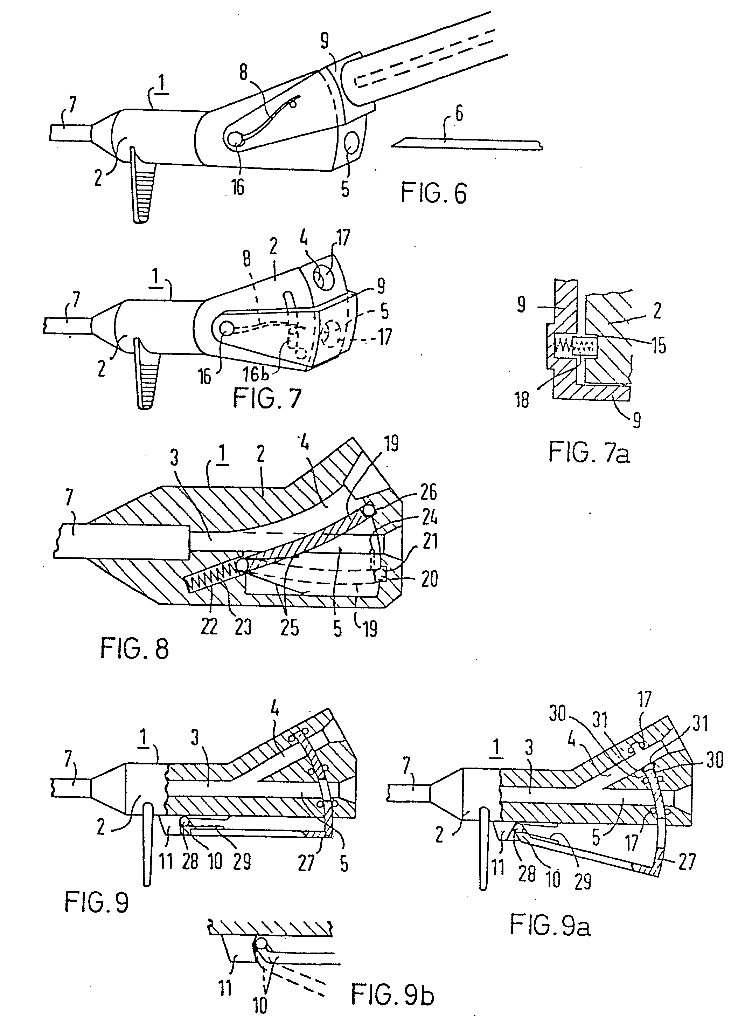

Der Katheteranschlußkopf 1 besteht im wesentlichen aus dem Grundkörper 2, der entweder nur mit einem einzigen geraden Kanal 3 (Fig. 1-5), oder mit zusätzlichen Kanälen 4 und 5 (Fig. 6 bis 10b), deren Achsen zueinander die Form eines « Y » beschreiben, versehen ist. In den Fig. 1 und 2 ist die Punktionskanüle 6 durch die mit dem Grundkörper 2 fest verbundene Kathetereinführröhre 7 soweit durchgeschoben, daß die Punktionskanüle 6 in an sich bekannter Weise um ein kleines Stück aus der Kathetereinführröhre 7 herausragt. Wenn der Punktionsvorgang beendet ist, wird die Punktionskanüle 6 herausgezogen, wodurch der Weg für das unter der Wirkung der Feder 8 stehende Anschluß- und Verschlußstück 9 freigegeben wird. Letzteres schwenkt soweit aus, bis seine Nase 10 gegen einen Anschlag 11 am Grundkörper 2 (Fig. 3) stößt und dabei gleichzeitig den Haltestift 12 für den Verriegelungsstift 13 aus seiner Haltelage verschiebt und den Weg für den Verriegelungsstift 13 freigibt, der alsdann unter der Wirkung der Feder 14 in die im Anschluß- und Verschlußstück 9 oder in dessen Nase 10 vorgesehene Ausnehmung 15 einrastet (Fig. 4a). Hierdurch wird bewirkt, daß das Anschluß- und Verschlußstück 9 nicht wieder aus dieser Lage zurückgeführt werden kann. Damit durch das in der Strecklage liegende Anschluß- und Verschlußstück 9 (Fig. 3 und 4) auch nicht versehentlich noch einmal eine Punktionskanüle 6 eingeführt bzw. zur Wirkung gebracht werden kann, weist dieses Anschluß- und Verschlußstück 9 entweder eine entsprechende Länge auf oder es ist mit einem Aufnahmekonus versehen, der für Punktionskanülen nicht paßt, so daß letztere nicht daran befestigt werden können.

Das Anschluß- und Verschlußstück 9 ist um die Achse 16 schwenkbar und es ist als zweiarmiger Hebel ausgebildet, wobei der eine Hebelarm gleichzeitig als Feder 8 ausgebildet sein kann, während der andere Hebelarm das eigentliche Anschluß- und Verschlußstück 9 darstellt, wie dies in den Fig. 1-5 dargestellt ist.

Die Abdichtung des Anschluß- und Verschlußstückes 9 gegenüber dem Grundkörper 2 erfolgt über Dichtungen bekannter Art, insbesondere über Ringdichtungen 17, wie sie in den Fig. 2a, 4a und 5 angedeutet sind. Diese Dichtungen bewirken, daß keine Außenluft und auch keine Schmutzteilchen in die Blutbahn gelangen können.

Wie aus den Fig. 6, 7 und 7a zu ersehen ist, kann das schwenkbare Anschluß- und Verschlußstück 9 auch als einarmiger Hebel ausgebildet sein. Während gemäß Fig. 6 das Anschluß- und Verschlußstück 9 durch eine außenliegende Feder 8 betätigt wird, kann dies gemäß Fig. 7 auch durch eine innenliegende und somit abgedeckte Feder 8 erfolgen. Ebenso kann in beiden Fällen die Verriegelung mit einem in Fig. 7a vergrößert dargestellten Federbolzen 18 durchgeführt werden, der in eine Ausnehmung 15 im Grundkörper 2 einrastet. Auf diese Weise kann die Anbringung eines besonderen Anschlages am Grundkörper 2 und die Anbringung einer Nase 10 am Anschluß- und Verschlußstück 9 entfallen. Das innenliegende Federelement gemäß Fig. 7 ist so angeordnet, daß es mit seinem einem Ende mit der Schwenkachse 16 verbunden ist, während sein anderes Ende an einem Anschlag in einem Langloch 16b geführt ist.

In Fig. 8 ist ein Katheteranschlußkopf dargestellt, bei dem das den Kanal 5 nach dem Punktionsvorgang verschließende Glied mit 19 bezeichnet ist. Bis zur Einführung der zeichnerisch nicht dargestellten Punktionskanüle liegt das Verschlußelement 19 mit der in die Ausnehmung 20 einrastenden Rolle oder Kugel 21 in der gestrichelt gezeichneten Lage, in der es durch die Wirkung der in der Bohrung 22 angeordneten Druckfeder 23 arretiert wird. Erst dadurch, daß die Punktionskanüle in den Kanal 5 eingeführt wird, drückt diese gegen den am Verschlußglied 19 angebrachten Mitnehmer 24 und entriegelt dadurch das Verschlußelement 19. Sobald dann der Punktionsvorgang beendet und die Punktionskanüle aus dem Kanal 5 zurückgezogen ist, schnellt das Verschlußelement 19 unter der Wirkung der Feder 25 in die mit ausgezogenen Linien dargestellte Lage und wird hier durch die Rolle oder Kugel 21 in der Ausnehmung 26 arretiert. Abgesehen davon, daß nunmehr der Kanal 5 für eine nochmalige Einführung einer Punktionskanüle für immer versperrt ist, dient das Verschlußelement 19 gleichzeitig als Teil der Wandung für den Kanal 4, so daß durch diesen nunmehr beispielsweise ein Katheter ohne weiteres einführbar ist.

In den Fig. 9, 9a und 9b ist als Verschlußelement für die Kanäle 4 und 5 ein Schieber 27 vorgesehen, der um den Punkt 28 verschwenkbar ist und unter der Wirkung der Feder 29 steht. Durch Anbringen der Einführöffnung im Schieber 27 in Höhe des in Fig. 9 dargestellten Kanals 5 ist es auch möglich, den Punktionsvorgang in dieser Schieberstellung durchzuführen. Die Abdichtung des Schiebers 27 erfolgt auch hier beispielsweise durch Ringdichtungen 17. Die Verriegelung des Schiebers 27 kann in einfachster Weise durch eine oder mehrere spreizbare Blattfeder/Blattfedern 30 erfolgen, die beispielsweise an der Schieberspitze befestigt ist/sind und sich beim Zurückziehen des Schiebers 27 gegen die Hinterschneidungen 31 legt/legen, wie dies in Fig. 9a dargestellt ist. Mit 10 ist wiederum eine Nase bezeichnet, die in der Einraststellung des Schiebers 27 an dem Anschlag 11 anliegt, wie dies insbesondere Fig. 9b zeigt.

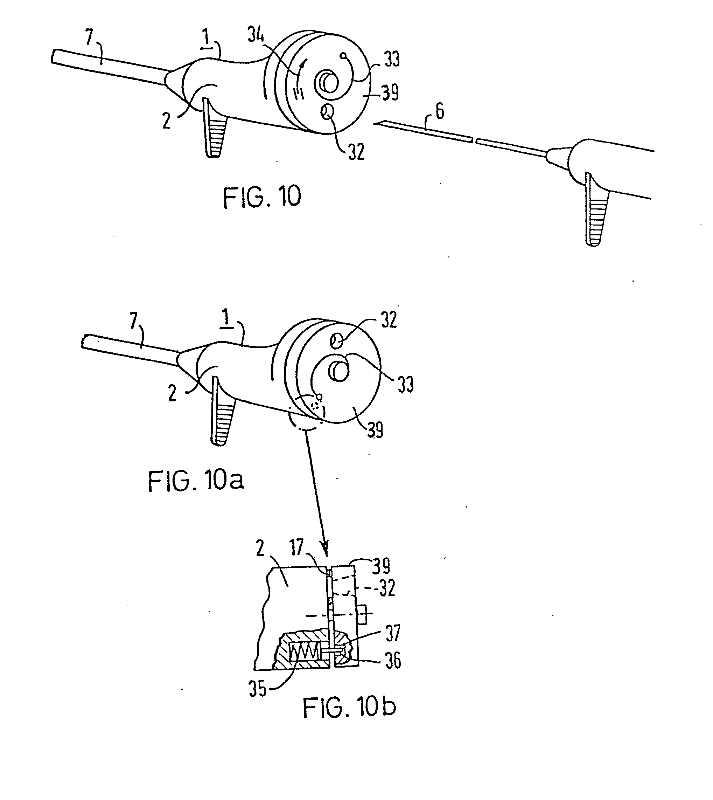

In den Fig. 10, 10a und 10b ist das Verschlußelement als drehbare Scheibe 39 mit einer exzentrisch angebrachten Bohrung 32 ausgebildet. In Fig. 10 ist die Spiralfeder 33 gespannt und das Verschlußelement in Form einer Scheibe 39 kann in gleicher Weise wie das in Fig. 7a dargestellte und mit dem Bezugszeichen 9 versehene Verschlußelement verriegelt sein. Beim Einführen der Punktionskanüle 6 wird die Verriegelung aufgehoben und nach zurückgezogener Punktionskanüle 6 dreht sich die Scheibe unter der Wirkung der Spiralfeder 33 in Richtung des Pfeiles 34. Dabei rastet der unter der Wirkung der Druckfeder 35 stehende Bolzen 36 in die im Verschlußelement 39 angebrachte Ausnehmung 37 automatisch ein (Fig. 10b), so daß der Weg für eine Punktionskanüle 6 auch bei dieser Ausführungsart für immer verschlossen ist und alle Gefahren, die den eingangs erwähnten, bekannten Geräten anhaften, beseitigt sind.

Die Erfindung zeichnet sich gegenüber bekannten Katheteranschlußköpfen durch eine Vielzahl von Vorteilen aus, die insbesondere in einer einfacheren und wesentlich schnelleren Bedienbarkeit liegen, was durch die ständige Bereitschaftsstellung des Anschluß- und Ver-

schlußstückes begründet ist. Darüber hinaus ist sichergestellt, daß bei der Umstellung von der Punktions- auf die Katheterfunktion keine Luftbläschen oder gar Schmutzteilchen in die Blutbahn gelangen können. Auch lassen sich Kunstfehler, wie das ungewollte « Punktieren » bereits liegender Katheter mit Sicherheit ausschließen.

**Ansprüche**

1. Katheteranschlußkopf (1) mit mindestens einem Kanal in einem Grundkörper (2) zum Anschluß eines Infusionssystems, Katheters oder dgl. und zum Einführen eines im wesentlichen aus einer Punktionskanüle (6) bestehenden Punktionsbestecks, wobei die Punktionskanüle (6) von einer flexiblen Kathetereinführröhre (7) umgeben ist, die von der Punktionskanüle (6) jedoch um ein vorbestimmtes Stück überragt wird, und wobei ferner die Punktionskanüle (6) nach dem Einführen der Kathetereinführröhre (7) in ein Blutgefäß aus dieser herausziehbar ist, und mit einem Verschluß- und Dichtungselement, das nach dem Zurückziehen der Punktionskanüle (6) den Punktionskanülenkanal (3, 5) automatisch abdichtet, dadurch gekennzeichnet, daß der für die Punktionskanüle (6) vorgesehene Kanal (3, 5) beim Zurückziehen der Punktionskanüle (6) durch Verschluß- (9, 19, 27, 39) und Verriegelungselemente (12-15 ; 15, 18 ; 20-26 ; 29-31 ; 35-37) automatisch derart verschlossen und verriegelt wird, daß ein Wiedereinführen der Punktionskanüle (6) nicht mehr möglich ist, und daß außer dem Punktionskanülenkanal (3, 5) auch gegebenenfalls vorhandene ein oder mehrere weitere Kanäle (4) gegenüber der Umwelt durch Dichtungselemente (17) automatisch abgedichtet werden.

2. Katheteranschlußkopf nach Anspruch 1, dadurch gekennzeichnet, daß der Grundkörper (2) als Träger für die Verschluß-, Verriegelungs- und Dichtungselemente dient.

3. Katheteranschlußkopf nach Anspruch 1 oder 2, dadurch gekennzeichnet, daß die Verschlußelemente (9) gleichzeitig als Anschlußstück für Infusionsgeräte und/oder Katheter ausgebildet sind (Fig. 1-7).

4. Katheteranschlußkopf nach Anspruch 3, dadurch gekennzeichnet, daß das als Anschlußstück dienende Verschlußelement (9) als zweiarmiger Hebel ausgebildet ist, dessen einer Hebelarm als Feder (8) für die Verschwenkung des Verschlußelementes (9) wirksam ist (Fig. 1-5).

5. Katheteranschlußkopf nach einem oder mehreren der Ansprüche 1-4, dadurch gekennzeichnet, daß das Verschlußelement (9) beim Verschwenken in seine Gebrauchslage eine Verriegelungseinrichtung (12, 13, 14) aus deren Haltelage verschiebt, wodurch ein Verriegelungsstift (13) freigegeben wird, der mit einer korrespondierenden Ausnehmung (15) verrastet und damit das Verschlußelement (9) in der Verriegelungslage festhält (Fig. 1-5).

6. Katheteranschlußkopf nach einem oder mehreren der Ansprüche 1-5, dadurch gekennzeichnet, daß das Verschlußelement (9) eine Nase (10) aufweist, die sich nach Entfernung der Punktionskanüle (6) unter der Wirkung der Feder (8) automatisch gegen einen die Verriegelungslage fixierenden Anschlag (11) legt (Fig. 1-5).

7. Katheteranschlußkopf nach Anspruch 1, dadurch gekennzeichnet, daß das Verschlußelement (9) als einarmiger Hebel mit einer von dem Verschlußelement (9) abgedeckten Feder (8) ausgebildet ist, die einerseits an der Schwenkachse (16) für das Verschlußelement (9) und andererseits in einem Langloch (16b) geführt ist (Fig. 6, 7 und 7a).

8. Katheteranschlußkopf nach Anspruch 7, dadurch gekennzeichnet, daß beim Verschwenken des Verschlußelementes (9) in die Verriegelungslage eine am Verschlußelement (9) angebrachte Verriegelungseinrichtung (18) mit einer korrespondierenden Ausnehmung (15) am Grundkörper (2) verrastet wird (Fig. 6, 7 und 7a).

9. Katheteranschlußkopf nach Anspruch 1, dadurch gekennzeichnet, daß das Verschlußelement (19) vor dem Punktionsvorgang eine zum Kanal (5) für die Punktionskanüle (6) im wesentlichen parallele Lage einnimmt, daß die Arretierung (20, 21) des Verschlußelementes (19) beim Herausziehen der Punktionskanüle (6) gelöst wird und damit das Verschlußelement (19) in eine den Kanal (5) für die Punktionskanüle (6) absperrende, höhere Arretierungslage gebracht wird, in der das Verschlußelement (19) gleichzeitig einen Teil der Wandung für einen weiteren Kanal (4) bildet (Fig. 8).

10. Katheteranschlußkopf nach Anspruch 1, dadurch gekennzeichnet, daß das Verschlußelement als L-förmiger Schieber (27) ausgebildet ist, dessen längerer Schenkel gelenkig mit dem Grundkörper (2) verbunden und dessen freies Ende des anderen kürzeren Schenkels mit einer oder mehreren spreizbaren Blattfedern (30) versehen ist, die sich beim Ausschwenken in die Verriegelungsstellung gegen Hinterschneidungen (31) im Grundkörper (2) legen (Fig. 9 und 9a).

11. Katheteranschlußkopf nach Anspruch 1, dadurch gekennzeichnet, daß das Verschlußelement als drehbare Scheibe (39) mit zur Scheibenachse exzentrischer Bohrung (32) für die Einführung der Punktionskanüle (6) ausgebildet ist, wobei beim Einführen der Punktionskanüle (6) in die Bohrung (32) die Verriegelung der Scheibe an dem Grundkörper (2) aufgehoben wird und nach dem Zurückziehen der Punktionskanüle (6) die Scheibe unter Federeinwirkung durch Verdrehung in die Schließ- und Verriegelungsstellung gebracht wird (Fig. 10, 10a und 10b).

12. Katheteranschlußkopf nach einem oder mehreren der Ansprüche 1-11, dadurch gekennzeichnet, daß in dem vom Grundkörper (2) und dem verschwenkbaren Verschlußelement (9, 27) gebildeten Spalt elastische Dichtungen (17), vorzugsweise Ringdichtungen, angeordnet sind (Fig. 5, 9 und 9a).

13. Katheteranschlußkopf nach einem oder mehreren der Ansprüche 1-9, 11 und 12, dadurch gekennzeichnet, daß die Verschlußelemente (9, 19, 39) beziehungsweise deren Nasen (10) oder deren Grundkörper (2) Ausnehmungen (15, 26, 37) aufweisen, in die in der Verriegelungsstellung des jeweiligen Verschlußelementes (9, 19, 39) Stifte, Bolzen, Rollen oder Kugeln (13, 18, 21, 36) einrasten (Fig. 4a, 7a, 8 und 10b).

## Claims

1. Catheter connection head (1) comprising at least one duct in a main body (2) to connect an infusion system, catheter or the like and to introduce a puncture instrument set consisting substantially of a puncture cannula (6), the puncture cannula (6) being enclosed by a flexible catheter inlet tube (7) beyond which the puncture cannula (6) projects by a predetermined amount, and the puncture cannula (6) upon the insertion of the catheter inlet tube (7) into a blood vessel being extractable therefrom, and comprising a closing and sealing element which, upon the withdrawal of the puncture cannula (6), automatically seals the puncture cannula duct (3, 5), characterized in that with the withdrawal of the puncture cannula (6) the duct (3, 5) provided for the puncture cannula (6) is automatically closed and sealed by closing (9, 19, 27, 39) and locking elements (12-15 ; 15, 18 ; 20-26 ; 29-31 ; 35-37) so that a reintroduction of the puncture cannula (6) is not possible any longer, and that the puncture cannula (3, 5) and in addition, one or several further ducts (4) if existing, are automatically sealed to the outside by sealing elements (17).

2. Catheter connection head according to claim 1, characterized in that the main body (2) serves as a carrier for the closing-, locking- and sealing elements.

3. Catheter connection head according to claims 1-2, characterized in that the closing elements (9) are simultaneously designed to serve as a connecting piece for the infusion devices and/or catheters (Figs. 1-7).

4. Catheter connection head according to claim 3, characterized in that the closing element (9) serving as a connecting piece is designed as a two-armed lever whose one lever arm is acting as a spring (8) for the swivelling of the closing element (9) (Figs. 1-5).

5. Catheter connection head according to one or more of the preceding claims 1-4, characterized in that the closing element (9), as it is swivelled into its operative position, displaces a locking means (12, 13, 14) from its stopping position, thus releasing a locking pin (13) which engages a corresponding recess (15) so that the locking element (9) is retained in the locking position (Figs. 1-5).

6. Catheter connection head according to one or more of the preceding claims 1-5, characterized in that the locking element (9) contains a nose (10) which, upon removal of the puncture cannula (6) and under the action of the spring (8) automatically abuts a stop (11) fixing the locking position (Figs. 1-5).

7. Catheter connection head according to claim 1, characterized in that the closing element (9) is a one-armed lever with a spring (8) covered by the closing element (9) the spring being guided, one the one hand, at the swivel axis (16) for the closing element (9) and, on the other hand, in an elongated hole (16b) (Figs. 6, 7 and 7a).

8. Catheter connection head according to claim 7, characterized in that with the swivelling of the closing element (9) into the locking position, a locking means (18) mounted at the closing element (9) engages a corresponding recess (15) at the main body (2) (Figs. 6, 7 and 7a).

9. Catheter connection head according to claim 1, characterized in that prior to the puncturing, the closing element (19) takes a position substantially in parallel to the duct (5) for the puncture cannula (6), that the stop (20, 21) of the closing element is released with the withdrawal of the puncture cannula (6) thus causing the locking element (19) to take a higher stop position locking the duct (5) for the puncture cannula (6) and in which the locking element (19) simultaneously forms part of the wall of another duct (4) (Fig. 8).

10. Catheter connection head according to claim 1, characterized in that the closing element is an L-shaped slider (27) whose longer leg is hinge-connected to the main body (2) while its free end of the other shorter leg is provided with one or more spreadable leaf springs (30) which, when swivelled out, are taking the locking position against undercuts (31) in the main body (2) (Figs. 9 and 9a).

11. Catheter connection head according to claim 1, characterized in that the closing element is a rotatable plate (39) with a bore (32) eccentric to the plate axis, for the introduction of the puncture cannula (6), the locking of the plate at the main body (2) being annulled with the introduction of the puncture cannula (6) into the bore (32) and, with the withdrawal of the puncture cannula (6), the plate being urged by torsion under spring action into the closing and locking position (Figs. 10, 10a, and 10b).

12. Catheter connection head according to one or more of the preceding claims 1-11, characterized in that flexible seals (17), preferably annular seals, are provided in the gap formed by the main body (2) and the swivelling closing element (9, 27) (Figs. 5, 9 and 9a).

13. Catheter connection head according to one or more of the preceding claims 1-9, 11 and 12, characterized in that the closing elements (9, 19, 39), and their noses (10) resp. or their main bodies (2) contain recesses (15, 26, 37) which, in the locking position of the respective closing element (9, 19, 39) are engaged by pins, bolts, rolls or balls (13, 18, 21, 36) (Figs. 4a, 7a, 8 and 10b).

## Revendications

1. Tête de raccord pour cathéter (1) avec au moins un conduit dans un corps de base (2), destinée au raccordement d'un système d'injection, d'un cathéter ou analogue, et à l'introduction d'un accessoire de ponction se composant essentiellement d'une canule de ponction (6), la canule de ponction (6) étant entourée par un tube flexible (7) d'insertion de cathéter, duquel la canule de ponction (6) dépasse cependant d'une distance prédéterminée, la canule de ponction (6) pouvant en outre, après introduction du tube d'insertion de cathéter (7) dans un vaisseau sanguin, être sortie de ce tube, la tête comportant également un élément d'obturation et d'étanchéité qui assure automatiquement l'obturation étanche du canal de canule de ponction (3, 5) après le retrait de la canule de ponction (6), caractérisée en ce que le canal (3, 5) prévu pour la canule de ponction (6) est automatiquement fermé et verrouillé, lors du retrait de la canule de ponction (6) par des éléments de fermeture (9, 19, 27, 39) et de verrouillage (12-15 ; 15,18 ; 20-26 ; 29-31 ; 35-37) de manière qu'une réinsertion de la canule de ponction (6) ne soit pas possible, et en ce que le canal de canule de ponction (3, 5) et, le cas échéant, un ou plusieurs autres canaux (4) sont aussi fermés automatiquement de façon étanche par rapport à l'environnement par des éléments d'étanchéité.

2. Tête de raccord pour cathéter selon la revendication 1, caractérisée en ce que le corps de base (2) sert de support pour les éléments de fermeture, de verrouillage et d'étanchéité.

3. Tête de raccord pour cathéter selon l'une des revendications 1 ou 2, caractérisée en ce que les éléments de fermeture (9) sont agencés simultanément comme organe de raccordement pour des appareils d'injection et/ou des cathéters (figures 1-7).

4. Tête de raccord pour cathéter selon la revendication 3, caractérisée en ce que l'élément de fermeture (9) servant d'organe de raccordement est agencé sous forme d'un levier à deux bras dont un des bras agit comme ressort (8) pour le pivotement de l'élément de fermeture (9) (figures 1-5).

5. Tête de raccord pour cathéter selon une ou plusieurs des revendications 1 à 4, caractérisée en ce que l'élément de fermeture (9) déplace, lors de son pivotement jusque dans sa position d'utilisation, un dispositif de verrouillage (12, 13, 14) à partir de sa position de retenue, ce qui libère un téton de verrouillage (13) qui vient se verrouiller dans un évidement correspondant (15) et qui maintient ainsi l'élément de fermeture (9) dans la position de verrouillage (figures 1-5).

6. Tête de raccord pour cathéter selon une ou plusieurs des revendications 1 à 5, caractérisée en ce que l'élément de fermeture (9) comporte un ergot (10) qui, après enlèvement de la canule de ponction (6) et sous l'action du ressort (8) vient s'appliquer automatiquement contre une butée (11) fixant la position de verrouillage (figures 1-5).

7. Tête de raccord pour cathéter selon la revendication 1, caractérisée en ce que l'élément de fermeture (9) est agencé sous forme d'un levier à un bras comportant un ressort (8) couvert par l'élément de fermeture (9) et qui est guidé d'un côté sur l'axe de pivotement (16) de l'élément de fermeture (9) et de l'autre côté dans un trou oblong (16b) (figures 6, 7 et 7a).

8. Tête de raccord pour cathéter selon la revendication 7, caractérisée en ce que, lors du pivotement de l'élément de fermeture (9) jusque dans la position de verrouillage, un dispositif de verrouillage (18) disposé sur l'élément de fermeture (9) est verrouillé à l'aide d'un évidement correspondant (15) sur le corps de base (2) (figures 6, 7 et 7a).

9. Tête de raccord pour cathéter selon la revendication 1, caractérisée en ce que l'élément de fermeture (19) occupe, avant le processus de ponction, une position essentiellement parallèle au canal (5) de la canule de ponction (6), et en ce que le système de blocage (20, 21) de l'élément de fermeture (19) est déverrouillé lors du retrait de la canule de ponction (6) et ainsi l'élément de fermeture (19) est amené dans une position d'arrêt plus haute qui obture le canal (5) pour la canule de ponction (6) et dans laquelle l'élément de fermeture (19) constitue simultanément une partie de la paroi d'un autre canal (4) (figure 8).

10. Tête de raccord pour cathéter selon la revendication 1, caractérisée en ce que l'élément de fermeture est agencé sous forme d'un poussoir (27) en forme de L, dont la branche longue est reliée de façon articulée au corps de base (2) et dont l'extrémité de l'autre branche courte est pourvue d'une ou plusieurs lames élastiques susceptibles de s'en écarter (30), et qui viennent se placer, lors du pivotement vers l'extérieur jusque dans la position de verrouillage, contre des parties en dépouille (31) du corps de base (2) (figures 9 et 9a).

11. Tête de raccord pour cathéter selon la revendication 1, caractérisée en ce que l'élément de fermeture est agencé sous forme d'un disque tournant (39) comportant un trou (32) d'insertion de la canule de ponction (6) qui est excentré par rapport à l'axe du disque, de sorte que, lors de l'insertion de la canule de ponction (6) dans le trou (32), le verrouillage du disque sur le corps de base (2) est supprimé et, après le retrait de la canule de ponction (6), le disque est amené sous l'action d'un ressort et par rotation dans la position de fermeture et verrouillage (figures 10, 10a et 10b).

12. Tête de raccord pour cathéter selon une ou plusieurs des revendications 1 à 11, caractérisée en ce qu'il est prévu, dans l'intervalle constitué par le corps de base (2) et l'élément de fermeture pivotant (9, 27) des joints d'étanchéité élastiques (17) de préférence des joints d'étanchéité annulaires (figures 5, 9 et 9a).

13. Tête de raccord pour cathéter selon une ou plusieurs des revendications 1 à 9, 11 et 12, caractérisée en ce que les éléments de fermeture (9, 19, 39) ou bien leurs ergots (10) ou bien le corps de base (2)/comportent des évidements (15, 26, 37) dans lesquels s'engagent des tétons, broches, rouleaux ou billes (13, 18, 21, 36) dans la position de verrouillage de l'élément de fermeture correspondant (9, 19, 39) (figures 4a, 7a, 8 et 10b).

0 029 864

FIG. 1

FIG. 2

FIG. 2a

FIG. 3

FIG. 4

FIG. 4a

FIG. 5

FIG. 6

FIG. 7

FIG. 7a

FIG. 8

FIG. 9

FIG. 9a

FIG. 9b

FIG. 10

FIG. 10a

FIG. 10b